# FASCICULE DE BREVET EUROPEEN

(11) **EP 1 545 435 B1**
(45) Date de publication et mention de la délivrance du brevet: **18.07.2007**
(21) Numéro de dépôt: 03775469.4
(22) Date de dépôt: 03.10.2003
(51) Int. Cl.: A61K 8/87, A61K 8/73, A61K 8/26, A61Q 1/00, A61Q 1/02

(54) **COMPOSITION DE MAQUILLAGE FILMOGENE**
FILMBILDENDE SCHMINKE
TOPICALLY-APPLIED FILM-FORMING COMPOSITION FOR MAKE-UP

(30) Priorité: 04.10.2002 FR 0212347
(43) Date de publication de la demande: 29.06.2005
(73) Titulaire: Laboratoires Lavipharm, 75116 Paris (FR)
(72) Inventeur: O'HALLORAN, David, US-Milltown, NJ 08850 (US); ZOLOTARSKY, Yelena, Springfield, NJ 07081 (US); FOTINOS, Spiros, 190 02 Peania (GR)
(74) Mandataire: Breese, Pierre
(86) Numéro de dépôt international: PCT/FR2003/002912
(87) Numéro de publication internationale: WO 2004/030606

(56) Documents cités:
- EP-A- 1 025 832
- WO-A-01/10397
- WO-A-02/39962
- FR-A- 2 814 674
- FR-A- 2 823 118
- US-B1- 6 306 411

## Description

L'invention concerne une nouvelle composition filmogène à usage topique pour le maquillage.

De nombreuses compositions cosmétiques ou thérapeutiques permettent d'appliquer au niveau de la peau des agents actifs cosmétiques ou thérapeutiques. Cependant, une application au niveau de la peau doit résister aux conditions environnementales et aux frottements avec les vêtements. Les compositions standard actuellement utilisées résistent mal et leur application doit être répétée pour obtenir l'effet désiré (maquillage). Dans le domaine du maquillage, les nombreuses compositions existantes ne permettent pas, aujourd'hui, de concilier le confort de la peau et la tenue de la couleur dans le temps. Ainsi, les films déposés lors des applications sont facilement éliminés par de simples frottements intervenant tout au long de la journée ou par des modifications d'état intervenant naturellement à la surface de la peau. Par exemple, en cosmétologie, l'utilisation de cellulose et d'argile pour mettre en suspension des pigments ou des particules est une pratique courante, mais le film produit est très facilement éliminé en frottant avec de l'eau. De plus, ce type de films est également très sec et très poudreux.

Les travaux de la Demanderesse ont permis de mettre au point une composition filmogène à usage topique pour colorer et/ou pigmenter la peau. Cette composition, lors de son application sur la peau, sèche pour donner un film distinctif, visible. Ce film présente également un aspect lisse uniforme. Il est confortable à porter, il résiste mieux aux conditions environnementales comme la pluie et aux frottements légers, comparé aux produits déjà connus dans l'art antérieur. Le présent film résiste également mieux à la transpiration et à la production de sébum.

Cette nouvelle composition filmogène à usage topique pour le maquillage est remarquable car elle constitue un nouveau mode de délivrance de pigments et colorants au niveau de la peau. En effet, grâce aux propriétés de résistance du film produit les ingrédients cosmétiques qu'elle contient restent intacts pendant un temps de contact avec la peau augmenté en vue d'un port prolongé.

Le polyuréthane-1 est un copolymère de monomères d'acide isophthalique, d'acide adipique, de glycol héxylène, de glycol néopentyl, d'acide diméthylolpropanique et de diisocyanate d'isophorone. Ce polymère est déjà connu pour sa capacité à former une pellicule. Il est notamment utilisé dans les produits fixants pour le soin des cheveux et dans le mascara. Les travaux de la Demanderesse ont notamment permis de mettre en évidence sa capacité à libérer au niveau de la peau des agents actifs. La cellulose est déjà utilisée pour sa capacité à former des pellicules et à libérer des agents actifs au niveau de la peau, mais n'a jamais été utilisée en combinaison avec le polyuréthane-1. Le polyuréthane-1 est de faible poids moléculaire, compris entre 10 000 et 15 000.

Le silicate de magnésium et d'aluminium est également utilisé comme stabilisateur et comme agent modifiant la viscosité, mais n'a jamais été associé dans une combinaison synergique avec le polyuréthane-1 et la cellulose.

Pris individuellement, chacun de ces composés a tendance à sécher et à s'écailler. Le maquillage mise au point par la Demanderesse est unique car elle présente une durée accrue, le film produit restant stable plus longtemps.

De plus, la pulvérisation de cellulose à partir d'un aérosol ou d'un pulvérisateur à pompe est généralement impossible. Avec la composition polymérique de l'invention, une brume uniforme est pulvérisée.

La composition filmogène à usage topique objet de l'invention est un système polymérique et est caractérisée en ce qu'elle contient au moins du polyuréthane-1, de la cellulose et du silicate de magnésium et d'aluminium. Comme indiqué précédemment, la composition filmogène selon l'invention est un nouveau système pour appliquer et fixer des pigments et/ou des colorants au niveau de la peau. Par conséquent, la composition de maquillage objet de l'invention contient en outre des colorants et/ou des pigments.

Les compositions de maquillage selon l'invention sont particulièrement adaptées pour un usage topique de façon à créer un film sur la peau. Elles peuvent de préférence être pulvérisées sur la peau. Aussi, les compositions de maquillage, objet de la présente invention, sont spécialement adaptées à une application sur le corps et, plus particulièrement, sur les jambes.

Les travaux de la Demanderesse ont également permis de mettre en évidence les proportions optimales des trois composés à utiliser dans le maquillage selon l'invention. Ainsi, le maquillage selon l'invention contient avantageusement :
- de 1 à 50 %, et de préférence de 3 à 30 % en poids de polyuréthane-1,
- de 0,01 à 2,0 % et de préférence de 0,01 à 1,0 % en poids de cellulose,
- de 0,05 à 5,0 % et de préférence de 0,1 à 3,0 % en poids de silicate de magnésium et d'aluminium.

En effet, c'est à partir d'une proportion de polyuréthane au moins égale à 3 % qu'on obtient un film particulièrement durable. Une proportion de cellulose inférieure ou égale à 1 % est particulièrement favorable aux propriétés de pulvérisation de la composition sans affecter les propriétés de résistance et de souplesse du film obtenu. Une proportion de silicate de magnésium et d'aluminium inférieure à 0,1 % diminue sensiblement l'efficacité de la mise en suspension des particules et une proportion supérieure à 3,0 % a tendance à rendre le film produit fragile.

La cellulose utilisée dans le maquillage selon l'invention peut être tout type de cellulose. De façon avantageuse, la cellulose contenue dans le maquillage selon l'invention est choisie parmi la méthylcellulose, l'hydroxypropyl méthylcellulose, l'éthylcellulose et l'hydroxyéthyl cellulose.

La composition de maquillage selon l'invention contient environ de 0,01 à 12 % de colorants et pigments.

Les colorants et pigments contenus dans la composition de maquillage objet de l'invention sont les pigments minéraux tel que Ti0₂ et Fe₂O₃, les pigments organiques, les laques, les pigments et colorants naturels, des nacres tels que les micas ou un mélange de ceux-ci. Dans un mode plus particulier de mise en oeuvre de l'invention, ces colorants et pigments sont choisis parmi les oxydes de fer, le dioxyde de titane et le mica titané.

La composition filmogène de maquillage selon l'invention peut contenir d'autres composés et notamment un ou plusieurs agents choisis parmi : les solvants hydrophiles, les solvants lipophiles, les humidifiants/plastifiants, les polymères épaississants autres que la cellulose et le polyuréthane-1, les colorants et pigments, les tensioactifs/émulsifiants, les parfums, les conservateurs, les agents chélatants, les absorbeurs d'UV, les antioxydants, les agents kératolytiques, le dihydroxy acétone et les agents favorisant la pénétration.

Les solvants hydrophiles utilisés dans le maquillage selon l'invention peuvent être tout solvant généralement utilisé en cosmétologie ou en dermatologie. On peut citer, à titre d'exemple et de façon non-limitative, comme solvant utilisable dans le maquillage selon l'invention, l'eau, des alcools comme l'éthanol, des glycols ou un mélange de ces composés. De façon avantageuse, le maquillage filmogène selon l'invention contient environ de 5 à 90 % en poids de solvants hydrophiles.

L'utilisation dans la composition selon l'invention de solvants lipophiles tels que des hydrocarbones ou des huiles est possible. On peut notamment citer à titre d'exemple et de façon non exhaustive, comme solvants lipophiles, des extraits végétaux, l'iso-paraffine, des huiles minérales, l'isododécane, l'huile d'amande douce ou d'autres huiles naturelles. De préférence, le maquillage selon l'invention contient environ de 1 à 10 % en poids de solvants lipophiles.

Les humidifiants/plastifiants utilisés dans le maquillage selon l'invention pour modifier les propriétés du film et sa durée peuvent être tout ingrédient liant l'eau généralement utilisé dans les produits cosmétiques ou dermatologiques tel que les glycols (glycérine, propylène glycol, 1,3-butylène glycol et polyéthylène glycols), sorbitol, sodium PCA, sodium citrate, diméthicone copolyol, cyclométhicone. Le maquillage selon l'invention contient avantageusement environ de 0,5 à 20 % d'humidifiants/plastifiants.

Les polymères épaississants autres que la cellulose et le polyuréthane-1 utilisés dans le maquillage selon l'invention pour modifier la viscosité, l'étalement, l'aspect du produit fini et la résistance du film peuvent être tout polymère ou tout épaississant généralement utilisé dans les produits cosmétiques ou dermatologiques comme, à titre d'exemple et de façon non-exhaustive, la gomme de xanthane, le PVA, le PVP, le carbomère ou des mélanges tels que le Simugel A (polyacrylate d'ammonium/isohexadecane/huile de castor PEG-40), Ceragel EZ-7 (polyacrylamide, polydecene, laureth-7), Sepigel 305 (polyacrylamide, C13-14 isoparaffine, laureth-7), Salcare SC-92 (polyquaternium 32, huile minérale). La proportion de polymères et d'épaississants autres que la cellulose et le polyuréthane-1 dans le maquillage selon l'invention est comprise entre 0,1 et 10 % par poids.

Les tensioactifs et les émulsifiants utilisés dans le maquillage selon l'invention pour stabiliser le produit fini ou pour améliorer son étalement sur la peau peuvent être tout émulsifiant ou tensioactif anionique, cationique ou non-ionique généralement utilisés dans les produits cosmétiques ou dermatologiques. Parmi ces tensioactifs et émulsifiants, on peut citer, à titre d'exemple et de façon non-limitative, les alcools éthoxylés, le sulfate de lauryl sodium, le polyquaternium-31. De façon avantageuse, le maquillage filmogène selon l'invention contient environ de 0,1 à 10 % de tensioactifs/émulsifiants.

Le maquillage selon l'invention peut avantageusement comprendre un parfum ou un mélange de parfums pour supporter un concept marketing ou pour masquer l'odeur naturelle de ladite composition. Le maquillage selon l'invention contient, de préférence, environ de 0,1 à 5 % de parfums.

Le maquillage selon l'invention peut également comprendre au moins un conservateur ou un mélange de conservateurs. Tout conservateur ou tout mélange de conservateurs connu de l'homme du métier dans le domaine des compositions cosmétiques ou dermatologiques est utilisable dans le maquillage de l'invention. L'utilisation d'un conservateur ou d'un mélange de conservateurs dans le maquillage selon l'invention permet de protéger ladite composition d'une contamination microbiologique. On peut notamment citer de façon non-exhaustive comme conservateur utilisable dans le maquillage le méthylparaben, le propylparaben, le phénoxyéthanol. Le maquillage selon l'invention contient avantageusement environ de 0,01 à 3 % de conservateurs.

Le maquillage selon l'invention peut également comprendre un agent chélatant ou un mélange d'agents chélatants pour lier les ions métalliques et pour aider à la stabilité globale de ladite composition. Tout agent chélatant utilisé dans les produits cosmétiques ou dermatologiques peut être ajouté à la composition comme, par exemple et de façon non-limitative, le EDTA, Na₂EDTA, Na₄EDTA. Le maquillage filmogène selon l'invention contient environ de 0,01 à 1 % d'agents chélatants.

Le maquillage selon l'invention peut comprendre des antioxydants afin de protéger ladite composition de l'oxydation. Tout oxydant ou mélange d'antioxydants généralement utilisé dans les produits cosmétiques ou dermatologiques est utilisable dans le maquillage selon l'invention. On peut notamment citer de façon non exhaustive comme antioxydant utilisable dans ladite composition le tocophérol, l'acétate de tocophérol, le gallate de propyle, le BHA ou le BHT. De façon avantageuse, le maquillage filmogène selon l'invention contient environ de 0,05 à 3 % en poids d'antioxydants.

Le maquillage selon l'invention se présente avantageusement sous une forme liquide ou sous une forme semi-liquide. De façon préférentielle, cette composition se présente sous la forme d'un spray, d'une lotion, d'un gel, d'un stick ou d'une mousse.

Lorsque le maquillage selon l'invention est appliqué sur la peau des utilisateurs, il peut être conservé pendant une période de 20 min à plusieurs heures, sous la forme d'un film sec. De façon avantageuse, le maquillage appliqué sur la peau des utilisateurs peut être éliminé avec de l'eau ou avec un mélange d'eau et de tensioactifs.

D'autres avantages et caractéristiques de l'invention apparaîtront à la lecture des exemples qui suivent concernant des formulations de compositions selon l'invention et leur procédé de préparation. Ces exemples sont donnés à titre illustratif et ne sauraient limiter la portée de l'invention.

### I. Exemples de formulation de compositions filmogènes à usage topique, objet de la présente invention.

Dans le tableau ci-dessous, sont présentées différentes formulations de compositions selon l'invention. Les formulations A et C correspondent à une composition de maquillage telle que définie précédemment contenant des oxydes de fer, du dioxyde de titane et des nacres avec du copolyol diméthicone et du cyclométhicone comme humidifiants/plastifiants. Les formulations B et D correspondent à une composition filmogène contenant du dioxyde de titane, des oxydes de fer et des nacres et du glycol comme humidifiants/plastifiants.

| **Ingrédients** | **A** % wt/wt | **B** % wt/wt | **C** % wt/wt | **D** % wt/wt |
|---|---|---|---|---|
| Eau | qsp 100 | qsp 100 | qsp 100 | qsp 100 |
| Hydroxypropyl méthylcellulose (Methocel E4M) | 0,32 | 0,20 | 0,32 | 0,50 |
| Silicate de Mg, A1 (Veegum HV) | 0,90 | 0,45 | 0,90 | 1,20 |
| Polyuréthane-1 (solution à 30% dans eau et alcool) | 18 | 12 | 18 | 25 |
| Carbomer | 0,15 | 0,10 | 0,15 | 0,20 |
| AMP (Aminométhyl propanol) | 0,20 | 0,20 | 0,20 | 0,30 |
| Glycol (butylène, propylène, hexalène ou glycérine ou PEG) | - | 2 | - | 4 |
| Dimethicone copolyol et cyclométhicone | 5 | - | 4 | - |
| Extraits biologiques | 0,70 | 1 | 0,70 | 2 |
| Conservateurs | 0,70 | 0,70 | 0,70 | 0,80 |
| Dioxyde de titane | 2,10 | 3,50 | 1,80 | 4 |
| Perle de mica (pigment nacré) | 0,20 | 0,10 | 1 | 0,50 |
| Oxyde de fer | 1,70 | 1,40 | 1,20 | 2,50 |

### II. Préparation de compositions de maquillage, objet de la présente invention.

Les polymères sont mélangés dans l'eau ou dans l'alcool ou dans un mélange de ceux-ci. Un apport de chaleur peut être nécessaire si de l'argile ou une gomme sont utilisées. Les polymères sont ajoutés l'un après l'autre et mélangés jusqu'au lissage du mélange en utilisant un mixeur standard à une vitesse choisie en fonction du mélange. Après avoir obtenu un mélange uniforme de polymères, les autres ingrédients sont ensuite ajoutés. Une grande vitesse de mélange doit être utilisée pour complètement incorporer les oxydes de fer et le dioxyde de titane dans la préparation.

### Référence:

International Cosmetic Ingredient Dictionary and Handbook, Eighth Edition
Harry's Cosmetology
Remington's Pharmaceutical Sciences

## Revendications

1. Maquillage filmogène à usage topique **caractérisée en ce qu'**il contient au moins du polyuréthane-1 de poids moléculaire allant de 10 000 à 15 000, de la cellulose, du silicate de magnésium et d'aluminium et des colorants et/ou des pigments, lesdits colorants et/ou pigments sont choisis parmi les pigments minéraux, les pigments organiques, les laques, les pigments et colorants naturels, des nacres tels que les micas, ou un mélange de ceux-ci à l'exclusion du dioxyde de titane, des oxydes de fer et des oxydes de zinc.

2. Maquillage selon la revendication 1, **caractérisée en ce qu'**il contient :
- de 1 à 50 %, et de préférence de 3 à 30 % en poids de polyuréthane-1,
- de 0,01 à 2,0% et de préférence de 0, 01 à 1,0 % en poids de cellulose,
- de 0,05 à 5,0 % et de préférence de 0,1 à 3,0 % en poids de silicate de magnésium et d'aluminium,
- de 0,01 à 12% de colorants et/ou pigments.

3. Maquillage selon la revendication 1 ou 2, **caractérisé en ce que** ladite cellulose est choisie parmi la méthylcellulose, l'hydroxypropyl méthylcellulose, l'éthylcellulose et l'hydroxyéthyl cellulose.

4. Maquillage selon l'une quelconque des revendications 1 à 3, **caractérisé en ce qu'**il contient en outre un ou plusieurs agents choisis parmi : les solvants hydrophiles, les solvants lipophiles, les humidifiants/plastifiants, les polymères épaississants autres que la cellulose et le polyuréthane-1, les colorants et pigments, les tensioactifs/émulsifiants, les parfums, les conservateurs, les agents chélatants, les antioxydants, les agents kératolytiques, le dihydroxy acétone et les agents favorisant la pénétration.

5. Maquillage selon la revendication 4, **caractérisé en ce qu'**il contient de 5 à 90 % en poids de solvants hydrophiles.

6. Maquillage selon l'une quelconque des revendications '4 ou 5, **caractérisé en ce qu'**il contient de 1 à 10 % en poids de solvants lipophiles.

7. Maquillage selon l'une quelconque des revendications 4 à 6, **caractérisé en ce qu'**il contient de 0,5 à 20 % d'humidifiants/plastifiants.

8. Maquillage selon l'une quelconque des revendications 4 à 7, **caractérisé en ce qu'**il contient de 0,1 et 10 % en poids de polymères et d'épaississants autres que la cellulose et le polyuréthane-1.

9. Maquillage selon l'une quelconque des revendications 4 à 8, **caractérisé en ce qu'**il contient de 0,1 à 10 % de tensioactifs/émulsifiants.

10. Maquillage selon l'une quelconque des revendications 4 à 9, **caractérisé en ce qu'**il contient de 0,1 à 5 % de parfums.

11. Composition selon l'une quelconque des revendications 4 à 7, **caractérisé en ce qu'**il contient de 0,01 à 3 % de conservateurs.

12. Composition selon l'une quelconque des revendications 4 à 11, **caractérisé en ce qu'**il contient de 0,01 à 1 % d'agents chélatants.

13. Composition selon l'une quelconque des revendications 4 à 12, **caractérisé en ce qu'**il contient de 0,05 à 3 % en poids d'antioxydants.

14. Maquillage selon l'une quelconque des revendications précédentes, **caractérisé en ce qu'**il se présente sous une forme liquide ou sous une forme semi-liquide.

15. Maquillage selon l'une quelconque des revendications précédentes, **caractérisée en ce qu'**il se présente sous la forme d'un spray, d'une lotion, d'un gel, d'un stick ou d'une mousse.

16. Maquillage selon l'une quelconque des revendications précédentes, **caractérisée en ce qu'**il peut être conservé sur la peau des utilisateurs pendant une période de 20 min à plusieurs heures.

17. Maquillage selon l'une quelconque des revendications précédentes, **caractérisée en ce qu'**il s'élimine de sur la peau des utilisateurs avec de l'eau ou un mélange d'eau et de tensioactifs.

18. Utilisation d'un maquillage selon l'une quelconque des revendications précédentes pour le corps et, plus particulièrement, pour les jambes.

## Claims

1. Film-forming makeup for topical use, **characterised in that** it contains at least polyurethane-1 with a molecular weight of from 10,000 to 15,000, cellulose, aluminium and magnesium silicate and colorants and/or pigments, said colorants and/or pigments are chosen from the mineral pigments, the organic pigments, the lakes, the natural pigments and colorants, nacres such as mica, or a mixture of these, excluding titanium dioxide, iron oxides and zinc oxides.

2. Makeup according to claim 1, **characterised in that** it contains:
- from 1 to 50%, and preferably from 3 to 30% by weight of polyurethane-1,
- from 0.01% to 2.0%, and preferably from 0.01 to 1.0% by weight of cellulose,
- from 0.05 to 5.0%, and preferably from 0.1 to 3.0% by weight of aluminium and magnesium silicate,
- from 0.01 to 12% of colorants and/or pigments.

3. Makeup according to claim 1 or 2, **characterised in that** said cellulose is chosen among the methylcellulose, the hydroxypropyl methylcellulose, the ethylcellulose and the hydroxyethyl cellulose.

4. Makeup according to any one of claims 1 to 3, **characterised in that** it further contains one or more agents chosen among: the hydrophilic solvents, the lipophilic solvents, the moisturisers/plasticizers, the thickening polymers other than cellulose and polyurethane-1, the colorants and pigments, the surfactants/emulsifiers, the fragrances, the preservatives, the chelating agents, the antioxidants, the keratolytic agents, the dihydroxyacetone and the penetration-promoting agents.

5. Makeup according to claim 4, **characterised in that** it contains from 5 to 90% by weight of hydrophilic solvents.

6. Makeup according to any one of claim 4 or 5, **characterised in that** it contains from 1 to 10% by weight of lipophilic solvents.

7. Makeup according to any one of claims 4 to 6, **characterised in that** it contains from 0.5 to 20% moisturisers/plasticizers.

8. Makeup according to any one of claims 4 to 7, **characterised in that** it contains from 0.1 and 10% by weight of polymers and thickeners other than cellulose and polyurethane-1.

9. Makeup according to any one of claims 4 to 8, **characterised in that** it contains from 0.1 to 10% surfactants/emulsifiers.

10. Makeup according to any one of claims 4 to 9, **characterised in that** it contains from 0.1 to 5% fragrances.

11. Composition according to any one of claims 4 to 7, **characterised in that** it contains from 0.01 to 3% preservatives.

12. Composition according to any one of claims 4 to 11, **characterised in that** it contains from 0.01 to 1% chelating agents.

13. Composition according to any one of claims 4 to 12, **characterised in that** it contains from 0.05 to 3% by weight of antioxidants.

14. Makeup according to any one of the previous claims, **characterised in that** it is in a liquid form or semiliquid form.

15. Makeup according to any one of the previous claims, **characterised in that** it is in the form of a spray, a lotion, a gel, a stick or a foam.

16. Makeup according to any one of the previous claims, **characterised in that** it can be retained on the users' skin for a period of 20 min. to several hours.

17. Makeup according to any one of the previous claims, **characterised in that** it is removed from the users' skin with water or a mixture of water and surfactants.

18. Use of a makeup according to any one of the previous claims for the body and, more particularly, for the legs.

## Patentansprüche

1. Filmbildende Schminke zur lokalen Anwendung, **dadurch gekennzeichnet, dass** sie mindestens Polyurethan-1 eines Molekulargewichts zwischen 10000 und 15000, Zellulose, Magnesiumsilikat und Aluminium und Farbstoffe und/oder Pigmente enthält, wobei diese Farbstoffe und/oder Pigmente unter mineralischen Pigmenten, organischen Pigmenten, Lacken, natürlichen Pigmenten und Farbstoffen, Perlmuttern wie Glimmer oder eine Mischung davon ausgewählt sind, jedoch Titandioxid, Eisenoxide und Zinkoxide ausgeschlossen sind.

2. Schminke nach Anspruch 1, **dadurch gekennzeichnet, dass** sie Folgendes enthält:
- 1 bis 50, vorzugsweise 3 bis 30 Gewichtsprozent Polyurethan-1,
- 0,01 bis 2,0, vorzugsweise 0,01 bis 1,0 Gewichtsprozent Zellulose,
- 0,05 bis 5,0, vorzugsweise 0,1 bis 3,0 Gewichtsprozent Magnesium- und Aluminiumsilikat,
- 0,01 bis 12 Prozent Farbstoffe und/oder Pigmente.

3. Schminke nach Ansprüchen 1 oder 2, **dadurch gekennzeichnet, dass** die Zellulose unter Methylzellulose, Hydroxypropylmethylzellulose, Ethylzellulose und Hydroxyethylzellulose ausgewählt ist.

4. Schminke nach einem der Ansprüche 1 bis 3, **dadurch gekennzeichnet, dass** sie außerdem eines oder mehrere der nachfolgenden Mittel enthält: wasseraufsaugende Lösungsmittel, fettaufsaugende Lösungsmittel, Befeuchter / Plastifizierer, verdickenden Polymere außer Zellulose und Polyurethan-1, Farbstoffe und Pigmente, Tenside / Emulsionsbildner, Parfums, Konservierungsmittel, Chelatbildner, Oxidationshemmer, keratolytische Mittel, Dihydroxyazetone sowie das Eindringen fördernde Mittel.

5. Schminke nach Anspruch 4, **dadurch gekennzeichnet, dass** sie 5 bis 90 Gewichtprozent wasseraufsaugende Lösungsmittel enthält.

6. Schminke nach einem der Ansprüche 4 oder 5,**dadurch gekennzeichnet, dass** sie 1 bis 10 Gewichtprozent fettaufsaugende Lösungsmittel enthält.

7. Schminke nach einem der Ansprüche 4 bis 6,**dadurch gekennzeichnet, dass** sie 0,5 bis 20 Prozent Befeuchter / Plastifizierer

8. Schminke nach einem der Ansprüche 4 bis 7,**dadurch gekennzeichnet, dass** sie 0,1 1 bis 10 Gewichtprozent Polymere und Verdicker außer Zellulose und Polyurethan-1 enthält.

9. Schminke nach einem der Ansprüche 4 bis 8,**dadurch gekennzeichnet, dass** sie 0,1 bis 10 Prozent Tenside / Emulsionsbildner enthält.

10. Schminke nach einem der Ansprüche 4 bis 9,**dadurch gekennzeichnet, dass** sie 0,1 bis 5 Prozent Parfüm enthält.

11. Zusammensetzung nach einem der Ansprüche 4 bis 7, **dadurch gekennzeichnet, dass** sie 0,01 bis 3 Prozent Konservierstoffe enthält.

12. Zusammensetzung nach einem der Ansprüche 4 bis 11, **dadurch gekennzeichnet, dass** sie 0,01 bis 1 Prozent Chelatbildner enthält.

13. Zusammensetzung nach einem der Ansprüche 4 bis 12, **dadurch gekennzeichnet, dass** sie 0,05 bis 3 Gewichtprozent Oxidationshemmer enthält.

14. Schminke nach einem der vorhergehenden Ansprüche, **dadurch gekennzeichnet, dass** sie in flüssiger oder halbflüssiger Form vorliegt.

15. Schminke nach einem der vorhergehenden Ansprüche, **dadurch gekennzeichnet, dass** sie in Form eines Sprays, einer Lotion, eines Gels, eines Sticks oder eines Schaums vorliegt.

16. Schminke nach einem der vorhergehenden Ansprüche, **dadurch gekennzeichnet, dass** sie während einer Dauer von 20 Minuten bis zu mehreren Stunden auf der Haut der Anwender gelassen werden kann.

17. Schminke nach einem der vorhergehenden Ansprüche, **dadurch gekennzeichnet, dass** sie mit Wasser oder einer Mischung aus Wasser und Tensiden von der Haut der Anwender gespült werden kann.

18. Anwendung einer Schminke nach einem der vorhergehenden Ansprüche auf dem Körper und insbesondere auf den Beinen.
